(19) Europäisches Patentamt

European Patent Office

Office européen des brevets

(11) **EP 1 683 572 A1**

(12) **EUROPEAN PATENT APPLICATION**
published in accordance with Art. 158(3) EPC

(43) Veröffentlichungstag:
26.07.2006 Patentblatt 2006/30

(21) Anmeldenummer: 04788527.2

(22) Anmeldetag: **20.09.2004**

(51) Int Cl.:
*B01J 20/02* (2006.01)    *B01J 20/06* (2006.01)
*B01J 20/08* (2006.01)    *B01J 20/10* (2006.01)
*B01J 20/24* (2006.01)    *B01J 20/26* (2006.01)
*B01J 20/30* (2006.01)

(86) Internationale Anmeldenummer:
**PCT/RU2004/000366**

(87) Internationale Veröffentlichungsnummer:
**WO 2005/035113 (21.04.2005 Gazette 2005/16)**

(84) Benannte Vertragsstaaten:
**AT BE BG CH CY CZ DE DK EE ES FI FR GB GR HU IE IT LI LU MC NL PL PT RO SE SI SK TR**

(30) Priorität: **14.10.2003 RU 2003130213**

(71) Anmelder:
• **Kutushov, Mikhail Vladimirovich**
**Moscow, 125414 (RU)**

• **Germanov, Evgeny Pavlovich**
**Moscow, 121085 (RU)**

(72) Erfinder: **KUTUSHOV, Mikhail Vladimirovich**
**Moscow, 125414 (RU)**

(74) Vertreter: **Jeck, Anton**
**Patentanwalt,**
**Klingengasse 2**
**71665 Vaihingen/Enz (DE)**

(54) **MAGNETISCH BETÄTIGTES ABSORPTIONSMITTEL UND HERSTELLUNGSVERFAHREN DAFÜR**

(57)    Die Erfindung betrifft die Biologie und die Medizin und kann zur Reinigung biologischer Fluide und dazu verwendet werden, den Inhalt der Fluide in Übereinstimmung mit physiologischen Standards zu bringen. Der Sinn dieser Erfindung liegt darin, dass das Absorbens einen ferromagnetischen Kern aufweist, der mit einer ein- oder zweischichtigen Schale versehen oder nicht versehen ist, und dass dieser Kern in Form einer Platte verkörpert ist, deren Größe in der Ebene im Bereich von 500-5000 $\mu$m und in der Stärke im Bereich von 0,1-1000 $\mu$m liegt. Das Verfahren gemäß der Erfindung zur Herstellung des magnetischen Absorptionsmittels besteht im Verdampfen und/oder Schmelzen eines Magnetmaterialpulvers in einem Niedrigtemperaturplasma, im Abkühlen und Kondensieren des so erhaltenen Verdampfungs- und/oder Schmelzpartikelprodukts in einem Gasstrom, im Überführen des kondensierten Produkts in Form von Kristallen oder Mikroperlen entsprechender Metalle, in entsprechender Weise zu einem Stabilisator enthaltenden Dispersionsmedium, und im Aufrechterhalten dieses Mediums, bis eine Gasfreisetzung vorüber ist.

Danach werden diese Kristalle oder Mikroperlen durch Abplatten weiterverarbeitet, beispielsweise durch Pressen in solcher Weise, dass Platten einer bestimmten Stärke erhalten werden. Diese Platten werden wiederholt (bis zu zehnmal) in destilliertem Wasser gewaschen, wobei die schwachen Plattenabschnitte **dadurch** abgetrennt werden, dass sie in Wasser beispielsweise der Wirkung einer Ultraschallquelle ausgesetzt werden, deren Leistung beispielsweise im Bereich von 200-300 W/cm$^2$ liegt. Die so hergestellten Platten werden getrocknet. Die getrockneten Platten werden aufgebrochen, die Absorptionskerne der erforderlichen Größe werden erhalten, und es werden Schicht auf Schicht daraus Schalen gebildet. Das Endprodukt wird in leicht geschützten, versiegelten Containern abgepackt und sterilisiert, beispielsweise durch $\gamma$-Strahlung. Das Endprodukt kann auch in Form eines Absorptionsmittels ausgewählt werden, das unmittelbar nach seiner Fraktionierung gewonnen wird.

EP 1 683 572 A1

**Beschreibung**

Die vorliegende Erfindung ist im Bereich der Biologie und Medizin angesiedelt und kann zur Abreinigung von Körperflüssigkeiten und zur Anpassung deren Zusammensetzung an die physiologischen Normen eingesetzt werden.

Aus SU 1589327 ist ein erstes magnetisches Sorbens (MGS) bekannt, welches aus Eisenkristallen in einer Größe von 10 bis 15 nm besteht (vgl. Urheberschein UdSSR 1589327, Priorität 14.04.1988, IPC C 01 G 49/08).

Das bekannte Sorbens übt eine bakterizide Wirkung aus, aber sein Einsatz ist begrenzt, weil es nur «in vitro» eingesetzt werden kann.

Den nächstliegenden Stand der Technik bildet ein aus RU 2178313 C1 bekanntes zweites MGS, welches aus sortierten Kernen mit einer Körnung von 0,1 bis 1000 $\mu$m besteht, die entweder aus Eisen oder aus Eisenoxiden, entweder aus Nickel oder aus Eisennickellegierung hergestellt und mit einer ein- oder zweischichtigen Beschichtung umhüllt sind. Die Beschichtung besteht entweder aus Kohlenstoff oder aus Aluminiumoxid, entweder aus Siliziumdioxid oder aus Zirkoniumdioxid, entweder aus Dextran (z.B. aus Sefadex) oder aus Gelatine, entweder aus Albumin oder aus Polysacharid (z.B. aus Stärke), entweder aus Austauschharzen (z.B. aus Kationen) oder aus Anionen, wobei die Außenschicht der Beschichtung Antikörper enthält. Die Außenschicht ist entweder mit einem Medikament oder medizinischen Wirkstoff, wie etwa einem Antibiotikums, oder mit Phthalohydrazidsalzen, wie etwa den Salzen von 5- Amino-2,3-Dihydro-1,4-Dion behandelt, oder als eine fermentierte Außenschicht hergestellt, beispielsweise durch Urease (vgl. z. B. Russ. Patent Nr. 2178313, Priorität vom 29.08.2000, IPC A 61 M 1/16).

Dieses Sorbens stellt ein wirksames Mittel bei der als Desoxidation bekannten Wiederherstellung der Körperflüssigkeiten bis auf die physiologischen Sollwerte dar, indem es die Reinigung beispielsweise von Blut von nieder-, mittel - und hochmolekularen Endotoxinen und Exotoxinen ohne Störung der Fließeigenschaften des Blutes, die Korrektur der Ferment- und Immunzusammensetzung von Körperflüssigkeiten, sowie die Vernichtung der pathogenen Mikroflora von Viren und Retroviren erlaubt. Da ein solches Sorbens teuer ist und für ein entsprechendes Heilverfahren eine große Menge dieses Sorbens benötigt wird, ist eine Behandlung unter Verwendung dieses Sorbens sehr kostspielig.

Aus SU 1589327 ist darüber hinaus ein Verfahren zu Herstellung des oben beschriebenen ersten MGS bekannt.

Dieses Herstellungsverfahren umfasst eine Verdampfung von Eisenpulver in einem kalten Plasma unter $10^4$ x und 0,5-5° C unter Argonatmosphäre. Das gewonnene dampfförmige Produkt wird scharf abgekühlt und im Argongasstrom kondensiert.

Anschließend wird das in Form von Kristallen ausgeschiedene Produkt in ein Dispergens übertragen, das einen Stabilisator enthält, beispielsweise Wasser mit einem pH-Wert von 7-9 oder ein Öl. Das Dispergens wird aufgerührt und wird für einen Zeitraum von 10 bis 15 Stunden bei einer Temperatur von 50 bis 90° C und einem Residualdruck von 1 bis 3 Millimeter Quecksilbersäule bis zum Abschluss der Gasabscheidung abgelagert.

Das bekannte Verfahren erlaubt die Herstellung eines Eisenteilchen in Form von Eisenkristallen von 10 bis 15 nm Größe umfassenden Sorbens. Dieses Sorbens weist jedoch wegen der kleinen Teilchengröße eine nur geringe magnetische Suszeptibilität, weshalb zur Ausscheidung des Sorbens aus einem biologischen Medium Magnetfelder mit einer Stärke von 1 bis 3 Tesla nötig sind. Dies ist den medizinischen Normen nach unzulässig.

Aus RU 2109522 C1 ist ein Verfahren zur Herstellung des oben beschriebenen zweiten MGS bekannt. Das Verfahren umfasst eine Fraktionierung eines hochdispersen Pulvers *Ferram reductum* in einem Inertgasstrom mit einer Geschwindigkeit von 0,02 bis 1,00 m/s unter Einwirkung eines Magnetfelds mit einer Stärke von 10 bis $10^3$ A/m sowie einer nachfolgenden Wärmebehandlung der gewonnenen Eisenteilchen bei einer Temperatur von 1000 bis 1500° C im Inertgasstrom, der Kohlenmikroteilchen und/oder Kieseloxidmikroteilchen und/oder Aluminiumoxidmikroteilchen enthält. Anschließend wird die Oberfläche der Eisenteilchen des Sorbens mit biologisch aktiven Verbindungen eingehüllt, entweder mit Nahrungsmitteleiweißen oder mit Dextran, entweder mit einem Medikament oder medizinischen Wirkstoff oder mit Antikörpern.

Dieses Verfahren erlaubt die Herstellung eines MGS mit einer definierten chemischen Zusammensetzung, die bei Beseitigung «in vivo» oder «in vitro» von nieder-, mittel - und hochmolekularen Toxinen, Mikroflora und Retroviren wirksam ist. Das Verfahren ist durch die Möglichkeit der Gewinnung eines Sorbens mit dreidimensionalen Teilchen mit Abmessungen von 0,5 bis 2,5 $\mu$m mit überwiegend gleichen Abmessungen im Durchmesser und Dicke begrenzt, wobei das Sorbens eine der Form der Teilchen entsprechende Oberflächegröße aufweist.

Eine erste Aufgabe der Erfindung ist es deshalb, ein Sorbens zu entwickeln, das ähnlich dem oben beschriebenen zweiten Sorbens wirkt, aber eine wesentlich größere Teilchenoberfläche besitzt, wobei die Masse des Sorbens im Wesentlichen gleich bleiben soll.

Eine zweite Aufgabe der Erfindung ist es darüber hinaus ein Verfahren zur Herstellung eines solchen Sorbens mit größerer Teilchenoberfläche und im Wesentlichen unveränderter Masse zu entwickeln.

Die erste Aufgabe wird dadurch gelöst, dass der ohne oder mit einer ein- oder zweilagigen Beschichtung versehene ferromagnetische Kern des erfindungsgemäßen magnetischen Sorbens, plattenförmig ist und eine Flächenabmessung von 500 bis 5000 $\mu$m und eine Dicke von 0,1-1000 $\mu$m aufweist.

Der Kern besteht dabei vorzugsweise aus Eisen oder aus Eisenoxiden, aus Nickel oder aus einer Eisennik-

kellegierung, aus einer Eisen- oder Nickellegierung mit Titan oder aus einer Eisen- oder Nickellegierung mit Tantal, aus einer Eisennickeltitanlegierung oder aus einer Eisennickeltantallegierung.

Bei einer Ausführung des erfindungsgemäßen Sorbens mit einer einlagigen Beschichtung besteht diese vorzugsweise entweder aus Kohlenstoff oder aus Aluminiumoxiden, entweder aus Siliziumdioxid oder aus Zirkoniumdioxid, entweder aus Dextran, wie beispielsweise aus Sefadex, oder aus Gelatine, entweder aus Albumin oder aus Polysacharid, wie beispielsweise aus Stärke, aus Austauschharzen, wie beispielsweise aus Kationen oder Anionen.

Bei einer Ausführung des erfindungsgemäßen Sorbens mit einer doppellagigen Beschichtung besteht die dem Kern nächste, erste Schicht (Inneschicht) entweder aus Kohlenstoff oder aus Aluminiumoxiden, entweder aus Siliziumdioxid oder aus Zirkoniumdioxid, und die zweite Schicht (Außenschicht) entweder aus Dextran, wie beispielsweise aus Sefadex, oder aus Gelatine, entweder aus Albumin oder aus Polysacharid, wie beispielsweise aus Stärke, aus Austauschharzen, wie beispielsweise aus Kationen oder Anionen.

Vorzugsweise besteht die Außenschicht aus Antikörpern, oder enthält solche. Dabei ist denkbar, dass die Außenschicht mit einem Medikament oder einem medizinischen Wirkstoff, beispielsweise einem Antibiotikum oder einem Phthalohydrazidsalz, wie etwa einem Salz von 5- Amino-2,3-Dihydro-1,4-Dion behandelt, oder, beispielsweise durch Urease, als fermentierte Außenschicht erzeugt ist.

Die zweite Aufgabe wird dadurch gelöst, dass gemäß einem erfindungsgemäßen Verfahren zur Herstellung eines MGS zunächst ein pulverförmiges ferromagnetisches Material, beispielsweise Eisen- und/oder Nickelpulver, und/ oder Titan- und/oder Tantalpulver, in einem kalten Plasma bei einer Temperatur von $10^4 x0{,}5$ bis 5° C verdampft oder geschmolzen wird. Das gewonnene dampfförmige oder als geschmolzene Teilchen der entsprechenden Metalle oder der entsprechenden Metallengemische vorliegende Produkt wird dann scharf abgekühlt und in einem Gasstrom, beispielsweise in einem Argongasstrom, kondensiert. Danach wird das als Kristalle oder entsprechend als Legierungsmikroblöckchen von entsprechenden Metallen ausgeschiedene Produkt in ein Dispergens übertragen, das einen Stabilisator enthält, wie beispielsweise Wasser und/oder Öl. Das Dispergens wird nach und nach umgerührt und für 5 bis 15 Stunden bei einer Temperatur von 50 bis 90° C und beim Residualdruck von 1 bis 5 Millimeter Quecksilbersäule belassen, bis die Gasausscheidung aufhört. Danach werden die Kristalle oder Mikroblöckchen bearbeitet und beispielsweise durch Pressformung in einer Kugelmühle plattgedrückt, bis man Platten der gewünschten Dicke gewinnt. Die Platten werden danach vielfach, vorzugsweise bis zu 10 Mal, im destillierten Wasser gespült. Dann werden die schwachen Plattenteile abgetrennt, indem die Platten beispielsweise im Wasser beispielsweise der Wirkung einer Ultraschallquelle mit einer Leistung von beispielsweise 200 bis 300 W/cm$^2$ ausgesetzt werden. Die gewonnenen Platten werden danach beispielsweise in einer Trockenkammer bei einer Temperatur von 80 bis 110° C abgetrocknet. Anschließend werden die trockenen Platten zur Ausscheidung der Sorbenskerne gewünschter Größe entweder in einem Inertgasstrom mit einer Geschwindigkeit von 0,02 bis 1,00 m/s unter Einwirkung eines Magnetfelds mit einer Stärke von 5x10-10$^3$ T/m fraktioniert, oder beispielsweise durch Zentrifugieren aussortiert. Danach wird auf den ausgeschiedenen Platten die Beschichtung lageweise gebildet. Zum Schluss wird das so gewonnene Produkt in hermetisch geschlossene Lichtschutzbehälter abgepackt und beispielsweise durch Bestrahlung sterilisiert. Dabei kann vorzugsweise das Sorbens ausgewählt werden, das sofort nach dem Sortieren gewonnen wird.

Dabei wird die erste, dem Kern am nächsten liegende Lage der Beschichtung durch Wärmebehandlung der aussortierten Platten bei einer Temperatur von 1000 bis 1500° C in einem Inertgasstrom, beispielsweise einem Argonstrom gebildet, der Mikroteilchen bestehend entweder aus Kohlenstoff oder aus Siliziumoxid, entweder aus Aluminiumoxid oder aus Zirkoniumoxid enthält.

Darüber hinaus ist denkbar, die erste Lage der Beschichtung dadurch zu bilden, indem auf eine Suspension aussortierter Platten in einer auf 30 bis 80° C temperierten wässrigen Lösung von Dextran oder von Gelatine, oder von Albumin oder von Stärke für einen Zeitraum von 1 bis 10 Minuten mithilfe einer Ultraschallquelle eingewirkt wird. Diese Suspension wird dann bis auf eine Temperatur von 4 bis 10° C abgekühlt und der dadurch gewonnene Niederschlag mit Formalin vergossen. Der Niederschlag wird dabei während eines Zeitraums von 10 bis 40 Minuten unter gleichzeitigem Umrühren im Formalin abgelagert. Danach wird der Niederschlag bei einer Temperatur von 25 bis 50° C sorgfältig abgetrocknet und zermahlen und die so gewonnenen Sorbenskapseln in einem Magnetfeld aussortiert.

Außerdem kann die erste Lage der Beschichtung durch Zusetzen eines Austauschharzes, beispielsweise von Amberlit, in die auf 40 bis 60° C gewärmte Suspension der fraktionierten Platten im destillierten Wasser gebildet werden, indem diese Suspension anschließend bis auf eine Temperatur von 15 bis 30° C abgekühlt, und wasserlösliche Salpetersäure ($HNO_2$) dazugegeben wird. Die Suspension wird anschließend für 10 bis 15 Minuten abgelagert und dann bis zu einer Temperatur von 4 bis 10° C abgekühlt. Dabei wird ein Niederschlag ausgeschieden, der in einer physiologischen Lösung gespült und in einer wässrigen Lösung umfassend ein Base-Salz-Gemisch aus $NH_4$ OH und NH4 Cl gepuffert wird.

Die zweite Lage der Beschichtung kann ebenfalls unter Rühren gebildet werden, indem für einen Zeitraum von 1 bis 10 Minuten mit Ultraschall auf die Suspension der ferromagnetischen, mit der ersten Lage der Beschichtung versehenen Kerne in einer erwärmten wässrigen Lösung von Dextran, Gelatine, Albumin oder Stärke eingewirkt wird. Die erste Lage der Beschichtung der ferromagnetischen Kerne besteht dabei vorzugsweise aus Kohlenstoff, Siliziumoxid, Aluminiumoxid oder aus Zirkoniumoxid. Diese Suspension wird dann bis auf eine Temperatur von 4 bis 10° C abgekühlt, wobei der auftretende Niederschlag mit Formalin abgegossen wird, und für einen Zeitraum von 10 bis 40 Minuten im Formalin unter Umrühren belassen wird. Danach wird der Niederschlag bei einer Temperatur von 25 bis 50° C sorgfältig ausgetrocknet und zermahlen. Anschließend werden die gewonnenen, kapselartig zweilagig beschichteten, das Sorbens bildenden Kerne im Magnetfeld gefiltert.

Darüber hinaus ist denkbar, die zweite Lage der Beschichtung durch Zugabe eines Austauschharzes, beispielsweise von Amberlit, in die bis auf 40 bis 60° C erwärmte Suspension der ferromagnetischen, mit der ersten Lage der Beschichtung versehenen Kerne in destilliertem Wasser zu bilden. Die erste Lage der Beschichtung der ferromagnetischen Kerne besteht auch hierbei vorzugsweise aus Kohlenstoff, Siliziumoxid, Aluminiumoxid oder aus Zirkoniumoxid. Dann wird diese Suspension bis auf eine Temperatur von 15 bis 30° C abgekühlt und Albumin, beispielsweise als Molken dazugegeben und vermischt. Anschließend wird flüssige Salpetersäure ($HNO_2$) in den Molken dazugegeben und für 10 bis 15 Minuten abgelagert. Danach wird das Gemisch bis auf eine Temperatur von 4 bis 10° C abgekühlt, wobei ein Niederschlag ausscheidet, der aktiviert wird, indem dieser in einer Modifizierungsmittellösung für 1,5 bis 2 Stunden ablagert. Der Niederschlag wird anschließend in einer physiologischen Lösung gespült und bis zur Erreichung pH von 4,0 0,5 in der Wasserlösung des Gemisches von Base NH4 OH und von Salz NH4 Cl gepuffert.

Vorzugsweise wird als Modifizierungsmittel Natriumperiodat ($NalO_4$) oder Glutaraldehyd in einer $Na_2SO_4$ -Wasserlösung von 3 bis 10 % eingesetzt.

Darüber hinaus ist denkbar, die äußere Lage der Beschichtung mit Antikörpern zu versehen. Hierzu wird in eine wässrige Suspension das MGS mit einer ein- oder zweilagigen Beschichtung dazugegeben, dessen äußere Lage vorzugsweise aus Sefadex oder aus Albumin besteht. Die vorzugsweise Glutaraldehyd oder Natriumperiodat umfassende wässrige Suspension ist mit einem Serum, beispielsweise einem Blutserum, modifiziert, welches Antikörper enthält, die für das sorbierte Antigen, beispielsweise für das Antigen des systematischen Lupus erythematodes spezifisch sind. Diese Mischung wird in einer Pufferflüssigkeit mit pH von 6,5 bis 10 für einen Zeitraum von 1 bis 3 Stunden bei einer Temperatur von 15 bis 25° C abgelagert und vermischt. Anschließend wird in diese Mischung Natriumborhydrid dazugegeben, die Mischung auf eine Temperatur von 4 bis 10° C abgekühlt, vermischt und wiederholt während 1 bis 3 Stunden abgelagert. Dann wird der Niederschlag abgezogen, gepuffert und getrocknet.

Ebenfalls ist denkbar, die äußere Lage der Beschichtung während deren Herstellung mit einem Medikament oder mit einem medizinischen Wirkstoff zu modifizieren, indem die das MGS mit ein- oder zweilagig beschichteten Kernen Suspension in einer physiologischen Lösung bis zu einer Temperatur von 35 bis 70° C erwärmt. Dabei hat das MGS vorzugsweise eine äußere Lage aus beispielsweise Dextran oder Gelatine. In diese Lösung wird ein vorzugsweise pulverförmiges Medikament oder ein pulverförmiger medizinischer Wirkstoff, beispielsweise ein Antibiotikum, wie etwa Oxazillin dazugegeben. Diese Mischung wird bei der oben genannten Temperatur für einen Zeitraum von 0,5 bis 2,5 Stunden abgelagert, sorgfältig vermischt und bis auf eine Temperatur von 4 bis 10° C abgekühlt. Die Überausscheidungsflüssigkeit wird in einem Magnetfeld abgegossen, und die Ausscheidung mithilfe fließenden destillierten Wassers gespült und später getrocknet.

Vorzugsweise wird die äußere Lage der Beschichtung bei deren Erzeugung durch Vorlösung von Ureasekristalle in Makrodiol, beispielsweise in Dibenzo-18 Kraun 6 modifiziert. Diese Lösung wird mit einer das MGS mit einer Beschichtung beispielsweise aus Sefadex und destilliertes Wasser umfassenden Suspension vermischt. Diese Mischung wird für einen Zeitraum von 2 bis 5 Stunden bei einer Temperatur von 25 bis 40°C abgelagert und bis auf eine Temperatur von 4 bis 10° C abgekühlt. Danach wird Formaldehyd dazugegeben, und die Mischung wird wieder für 1 bis 3 Stunden abgelagert. Anschließend wird die Überausscheidungsflüssigkeit in einem Magnetfeldes abgegossen und die Ausscheidung getrocknet.

Darüber hinaus kann die äußere Lage der Beschichtung modifiziert werden, indem eine ein MGS mit einer äußeren Lage beispielsweise aus Dextran enthaltende wässrige Suspension bis zu einer Temperatur von 40 bis 70° C erwärmt wird. Die Suspension wird anschließend mit einem Zirkoniumsalzpulver, beispielsweise mit einem entsprechenden Phthalhydrozidsalz, vermischt. Auf diese Mischung wird mit Ultraschall mit einer Schallintensität von 50 bis 120 Volt/Zentimeter für einen Zeitraum von 1 bis 10 Minuten eingewirkt. Danach wird die gewonnene Mischung bis auf eine Temperatur von 4 bis 10° C abgekühlt und Formaldehyd dazugegeben. Die Mischung wird anschließend für 1 bis 3 Stunden abgelagert und umgerührt. Die Überausscheidungsflüssigkeit wird dann in einem Magnetfeldes abgegossen und die Ausscheidung getrocknet.

Das erfindungsgemäße magnetische Sorbens (MGS) umfasst Kerne mit einer ein-oder zweilagigen, den Kern umhüllenden Beschichtung, oder ohne eine solche Beschichtung. Zur Herstellung der Kerne wird ein Pulver aus ferromagnetischem Material, beispielsweise aus Eisen (Fe), seinen Oxiden ($Fe_2 O_3$ oder $Fe_3 O_4$), aus Nickel (Ni), aus Eisennickellegierungen, aus einer Eisenlegierung oder einer Nickellegierung mit Titan (Ti), aus einer Eisenlegierung oder Nickellegierung mit Tantal (Ta), oder aus Eisennickeltitanlegierung, oder aus Eisennickeltantaltitanlegierung oder aus anderen magnetisch empfindlichen Stoffen verwendet.

Zur Verwendung für das erfindungsgemäße MGS werden plattenförmige Teilchen mit Flächenabmessungen von 500 bis 5000 $\mu$m und mit einer Stärke von 0,1 bis 1000 $\mu$m ausgewählt.

Zur Herstellung der Kerne des erfindungsgemäßen MGS wird pulverförmiges Eisen und/oder Nickel und/oder Titan und/oder Tantal mit einer Teilchengröße von $10^2$ bis $10^5$ nm in einem kalten Plasma bei einer Temperatur von $10^4$x 0,5 bis 5° C verdampft und/oder abgeschmolzen. Das dampfförmige und/oder geschmolzene, flüssige Produkt der entsprechenden Metalle oder des entsprechenden Metallengemischs mit einer Konzentration von 0,1 bis 0,5 Vol. % wird scharf auf eine Temperatur von 50 bis 80° C abgekühlt und in einem Stoffumsetzer in einem Gasstrom, beispielsweise in einem Argonstrom kondensiert. (Vgl. SU 1589327). Die dabei in einer Menge von beispielsweise 0,05 bis 10 Milligramm entstehenden metallischen Kristalle oder Mikroblöckchen werden anschließend in ein Dispergens übertragen, das ein Stabilisierungsmittel enthält, beispielsweise 50 bis 500 Milliliter destilliertes Wasser mit einem pH-Wert von 7 bis 9 und/ oder ein Mineralöl, beispielsweise Weißöl, oder ein Pflanzenöl, beispielsweise Olivenöl oder Sanddornöl. Vorher wird dem Dispergens beispielsweise Ölsäure dazugegeben. Die Dispergens wird für einen Zeitraum von 5 bis 15 Stunden abgelagert und bei einer Temperatur von 50 bis 90° C und einem Residualdruck von 1 bis 5 Millimeter Quecksilbersäule umgerührt, bis die Gasabscheidung aufhört.

Danach werden die Kristalle und/oder Mikroblöckchen bearbeitet, bis Platten von gewünschter Größe gewonnen werden. Dies kann beispielsweise durch Pressformung in einer Kugelmühle erfolgen, wo die Kristalle und/oder Mikroblöckchen plattgedrückt werden. Die Platten werden dann mehrfach, vorzugsweise bis zu 10 Mal mit dem destilliertem Wasser gespült. Danach werden die schwachen Plattenteile abgetrennt, indem die Platten beispielsweise in einem Wasserbad der Einwirkung einer Ultraschallquelle mit einer Leistung von 200 bis 300 Volt/Zentimeter$^2$ ausgesetzt werden.

Der so gewonnene Stoff, der Platten mit unterschiedlichen Hauptabmessungen sowie abgeplatze Stücke umfasst, wird massenweise beispielsweise in einer Trockenkammer bei einer Temperatur von 80 bis 110° C getrocknet. Danach werden die getrockneten Platten entweder in einem Inertgasstrom mit einer Geschwindigkeit von 0,02 bis 1,00 m/s unter Einwirkung eines Magnetfelds mit der Feldstärke von 5 x (10-10$^3$) T/Meter oder durch Zentrifugieren aussortiert, wobei Platten gewünschter Größe zur Verwendung als Kerne des MGS ausgeschieden werden, die dann schichtweise beschichtet werden. Das fertige Produkt wird in hermetisch geschlossene Lichtschutzbehälter abgepackt und beispielsweise durch Bestrahlen sterilisiert. Dabei können zur Verwendung als Sorbens direkt die nach dem Sortieren gewonnenen Platten ausgewählt werden. Die Ausbeute an geeigneten Kernen beträgt nach der Fraktionierung 60 bis 75 %.

Zur Bildung einer ersten, dem Kern am nächsten liegenden Lage der Beschichtung werden die aussortierten Platten bei der Temperatur von 1000 bis 1500° C in einem Wärmeofen in einem Inertgasstrom, beispielsweise in einem Argonstrom, einer Wärmebehandlung unterzogen. Der Inertgasstrom enthält dabei Mikroteilchen aus Kohlenstoff (C), aus Siliziumoxid ($SiO_2$), aus Aluminiumoxid ($Al_2O_3$ oder $Al_3O_4$) oder aus Zirkoniumoxid ($ZrO_2$). Die Strömungsgeschwindigkeit des Inertgasstroms beträgt hierbei 0,02 bis 1,2 m/s. Die Qualität der ersten Lage der Beschichtung ist von der Strömungsgeschwindigkeit des Inertgasstroms, sowie von der Sättigung des Inertgases mit Mikroteilchen des Beschichtungsstoffes und von der Größe dieser Mikroteilchen abhängig. In den angeführten Beispielen beträgt die Dicke der auf solche Weise gewonnenen ersten Lage der Beschichtung 0,2 bis 50 $\mu$m. Die brauchbare Ausbeute an Sorbens beträgt hierbei 70 bis 85 %.

Zur Herstellung einer ersten Lage der Beschichtung durch Einhüllen der Kerne des MGS mit Stoffen wie beispielsweise Dextran, Gelatine, Albumin oder Stärke, wird eine die aussortierten Platten in einer Menge von 2 bis 20 Gramm in 10 bis 50 Milliliter destilliertem Wasser enthaltende Suspension mit 50 bis 100 Milliliter einer bis auf eine Temperatur von 30 bis 80° C erwärmten, Dextran, Gelatine, Albumin oder Stärke in einem Verhältnis von 50 bis 95 Vol. % enthaltenden Wasserlösung vermischt. Die Stoffe werden für 1 bis 10 Minuten einer Ultraschall mit einer Frequenz von 10 bis 15 Kilohertz und mit einer Intensität von 50 bis 120 Volt/Zentimeter emittierenden Ultraschallquelle, beispielsweise einem Ultraschallgenerator UZDH-2T, unter gleichzeitigem Umrühren ausgesetzt (vgl. SU 1684616), bis eine homogene Zusammensetzung vorliegt. Danach wird diese Suspension beispielsweise in einer Kühlanlage bis auf 4 bis 10° C abgekühlt. Der hierbei entstehende Niederschlag wird mit Formalin (Wasserlösung HCHO) vergossen, und für 10 bis 40 Minuten unter gleichzeitigem Umrühren abgelagert. Der gewonnene Niederschlag wird bei der Temperatur von 25 bis 50° C sorgfältig getrocknet und zermahlen. Das so gewonnenen Sorbens wird in einem Magnetfeld mit einer Stärke von 5 x (10-10$^3$) A/M, beispielsweise im Magnetfeld eines Permanentmagneten aus einer Samarium(8t)- Kobalt (Co) - Legierung, gefiltert.

Die Dicke der auf solche Weise gewonnenen Lage der Beschichtung beträgt 0,5 bis 3 mm. Die quantitative Ausbeute an Sorbens beträgt 85 bis 95 % der Ausgangsausbeute.

Zur Herstellung einer ersten Lage der Beschichtung unter Verwendung eines Austauschharzes werden beispielsweise 10 bis 25 Gramm Amberlit einer auf 40 bis 60° C erwärmten, fraktionierte Platten in einer Menge von 2 bis 5 Gramm auf 10 bis 100 Milliliter destilliertem Wassers umfassende Suspension dazugegeben. Danach wird die Mischung bis auf 15 bis 30° C abgekühlt und 1 bis 10 Vol. % wässrige Salpetersäure ($HNO_3$) dazugegeben. Dann wird das Ganze für 10 bis 15 Minuten abgelagert und wieder bis auf 4 bis 10° C abgekühlt. Dabei scheidet sich ein Niederschlag aus, der mit einer physiologischen Lösung ausgespült und bis pH 4,0±0,5 in einer die Base $NH_4OH$ UND $NH_4Cl$ enthaltenden wässrigen Lösung gepuffert wird. Die Dicke der auf solche Weise gewonnenen Lage der Beschichtung beträgt 0,2 bis 1 Millimeter. Die quantitative Ausbeute an Sorbens beträgt hierbei 90 bis 92 % von der Ausgangsausbeute.

Zur Herstellung einer zweiten Lage der Beschichtung durch Einhüllen eines Kerne mit einer ersten Beschichtung aus Kohlenstoff, Siliziumoxid, Aluminiumoxid oder Zirkoniumoxid umfassenden MGS mit solchen Stoffen wie Dextran, Gelatine, Albumin oder Stärke, wird eine 2 bis 20 Gramm mit einer ersten Lage der Beschichtung versehenen ferromagnetischen Kerne in 10 bis 50 Milliliter destilliertes Wasser umfassende Suspension für 1 bis 10 Minuten unter Einwirkung von Ultraschall mit einer Intensität von 50 bis 120 Volt/Zentimeter in 50 bis 100 Mililliter auf 30 bis 80° C erwärmten 50 bis 95 %igen Lösung aus Dextran (entweder Gelatine - oder Albuminlösung, oder Stärkelösung) und destilliertem Wasser im destillierten Wasser vermischt. Danach wird diese Suspension bis auf 4 bis 10° C abgekühlt. Der gewonnene Niederschlag wird mit Formalin vergossen. Der Niederschlag wird für 10 bis 40 Minuten im Formalin unter gleichzeitigem Umrühren abgelagert. Dann wird der Niederschlag bei 25 bis 50° C sorgfältig getrocknet und zermahlen. Das gewonnene kapselartige Sorbens wird in einem Magnetfeld mit einer Stärke von 5 x 10 bis $10^3$ A/m filtriert. Die Dicke der auf solche Weise gewonnenen Lage der Beschichtung beträgt 0,5 bis 3 Millimeter. Die quantitative Ausbeute des Sorbens beträgt hierbei 85 bis 95 % von der Ausgangsausbeute.

Zur Herstellung einer zweiten Lage der Beschichtung unter Verwendung eines Austauschharzes wird die die mit einer ersten Lage der Beschichtung aus Kohlenstoff, Siliziumoxid, Aluminiumoxid oder Zirkoniumoxid versehenen ferromagnetischen Teilchen in einer Konzentration von 0,2 bis 0,5 g in 10 bis 100 ml destilliertem Wasser umfassende Suspension auf 40 bis 60° C erwärmt. Hierzu werden beispielsweise 1 bis 2 Gramm Amberlit dazugegeben und die so gewonnene Mischung auf 15 bis 30° C abgekühlt. Eine 1 bis 10 % ige Salpetersäure ($HNO_3$) wird für 10 bis 15 Minuten abgelagert, dann bis auf 4 bis 10° C abgekühlt und der auftretende Niederschlag ausgeschieden. Der Niederschlag wird für 1,5 bis 2 Stunden einer Modifizierungsmittellösung abgelagert und anschließend in einer physiologischen Lösung gespült. Der Niederschlag wird bis 4,0±0,5 in einer die Base $NH_4OH$ und das Salz $NH_4C1$ umfassenden wässrigen Lösung gepuffert.

Dabei werden Natriumperoidat ($NalO_4$) oder Glutaraldehyd in einer 3 bis 10 %igen wässrigen Lösung von $Na_2SO4$ - als Modifizierungsmittel eingesetzt. Die Dicke der auf solche Weise gewonnenen Lage der Beschichtung beträgt 0,2 bis 1 Millimeter. Die quantitative Ausbeute des Sorbens beträgt 90-95 % von der Ausgangsausbeute.

Zur Herstellung einer mit Antikörpern versehenen äußeren Lage der Beschichtung ist erfindungsgemäße vorgesehen, ein Serum, wie etwa ein Blutserum, welches Sefadex oder Albumin enthält, in einer Konzentration von 1 bis 50 Milliliter je 100 bis 150 Milliliter Suspension in eine wässrige Suspension eines MGS mit einer ein- oder zweilagigen Beschichtung, wobei die äußere Lage beispielsweise mit Glutaraldehyd oder Natriumperoidat ($NalO_4$) modifiziert ist, dazuzugeben. Das Serum wird vorzugsweise in Form einer Pufferflüssigkeit mit pH 6,5-10 dazugegeben. Das Serum enthält die zu dem zu sorbierenden Antigen spezifischen Antikörper, wie etwa das dem Antigen systematischen Lupus erythematodes. Diese Mischung wird für 1 bis 3 Stunden bei einer Temperatur von 15 bis 25° C abgelagert und umgerührt, später wird Natriumborhydrid der Mischung zugesetzt, die bis auf 4 bis 10° C abgekühlt wird, und während 1 bis 3 Stunden wiederholt abgelagert und umgerührt wird. Der entstehende Niederschlag wird ausgezogen, gepuffert und abgetrocknet. Die Dicke der entsprechenden Sphärenschicht nimmt dabei um 0,2 bis 0,5 Millimeter zu. Die quantitative Ausbeute an Sorbens beträgt hierbei 92 bis 95 % von der Ausgangsausbeute.

Darüber hinaus kann die äußere Lage der Beschichtung mit einem Medikament oder einem medizinischen Wirkstoff modifiziert werden, indem eine ein MGS mit einer ein-oder zweilagigen Beschichtung und einer äußeren Lage beispielsweise aus Dextran oder Gelatine in einer Konzentration von 10 bis 20 g auf 50 ml destilliertes Wasser enthaltende wässrige Suspension auf 35 bis 70° C in einer physiologischen Lösung (0,9 % NaCl in destilliertem Wasser) erwärmt, und ein pulverförmiges Medikament oder einen pulverförmigen medizinischen Wirkstoff, beispielsweise ein Antibiotikum, wie etwa Oxazillin, in einer Konzentration von 1 bis 5 g auf 10 bis 50 ml Suspension zusetzt. Diese Mischung wird bei der oben angegebenen Temperatur für 0,5 bis 2,5 Stunden abgelagert und sorgfältig umgerührt. Später wird diese Mischung bis auf 4 bis 10° C abgekühlt und die Überniederschlagflüssigkeit in einem Magnetfeld mit einer Stärke von 5 x ($10-10^3$) A/M abgegossen. Der Niederschlag wird mit fließendem destilliertem Wasser ausgespült und später bei einer Temperatur von 25 bis 40° C getrocknet. Die Dicke der entsprechenden äußeren Lage der Beschichtung nimmt dabei um 0,01 bis 0,1 Millimeter zu. Die quantitative Ausbeute des Sorbens beträgt hierbei 90 bis 95 % von der Ausgangsausbeute.

Darüber hinaus kann die äußere Lage der Beschichtung bei deren Herstellung durch Vorauflösen von beispielsweise 1 bis 5 g Ureasekristalle in 10 bis 15 ml Makrodiol, beispielsweise Dibenzo-18 Kraun 6, modifiziert werden. Diese Lösung wird mit einer ein MGS mit einer beispielsweise aus Sefadex bestehenden Beschichtung in einer Konzentration von 10 bis 15 g Sorbens auf 50 bis 100 ml Wasser umfassenden Suspension in destilliertem Wasser vermischt. Die Mischung wird für 2 bis 5 Stunden bei 25 bis 40° C abgelagert und umgerührt. Später wird Formaldehyd in einer Konzentration von 25 bis 30 ml auf 100 ml Mischung zugesetzt. Die Mischung wird wiederholt für 1 bis 3 Stunden abgelagert und umgerührt. Anschließend wird die Überniederschlagflüssigkeit unter Einwirkung eines Magnetfelds mit einer Stärke von 5 x ($10-10^3$) A/M abgegossen. Der gewonnene Niederschlag wird bei 50 bis 85° C beispielsweise in einer Trockenkammer getrocknet. Die Dicke der Beschichtung nimmt dabei um 0,5 bis 1 Millimeter zu. Die quantitative Ausbeute des Sorbens beträgt 90 bis 95 % von der Ausgangsausbeute.

Außerdem kann die äußere Lage der Beschichtung bei deren Herstellung durch Erwärmung der ein MGS mit einer Beschichtung beispielsweise aus Dextran in einer Konzentration von 15 bis 20 g Sorbens auf 75 bis 100 ml destilliertes Wasser enthaltenden wässrigen Suspension bis auf 40 bis 70° C modifiziert werden. Die Suspension wird anschließend mit einem Zirkoniumsalz, beispielsweise einem entsprechenden Phthalhydrazidsalz, wie etwa 5- Amino- 2,3- Dihydro-1,4-Dion, vermischt. Diese Mischung wird dann für 1 bis 10 Minuten einer Ultraschall mit einer Frequenz von 15 bis 25 kHz und mit einer Intensität von 50 bis 120 Volt/Zentimeter2 emittierenden Ultraschallquelle ausgesetzt. Anschließend wird die Mischung auf 4 bis 10° C abgekühlt und es wird Formaldehyd in einer Konzentration von 25 bis 30 ml auf 100 ml Mischung zugesetzt. Das Ganze wird dann für 1 bis 3 Stunden abgelagert und umgerührt und anschließend die Überniederschlagflüssigkeit in einem Magnetfeld mit einer Stärke von 5 x $(10\text{-}10^3)$ A/M abgegossen. Der Niederschlag wird dann noch bei einer Temperatur von 25 bis 45° C getrocknet. Die Dicke der entsprechenden Lage der Beschichtung nimmt dabei um 0,01 bis 0,1 Millimeter zu. Die quantitative Ausbeute des Sorbens beträgt hierbei 90 bis 95 % von der Ausgangsausbeute.

Die Verwendung des nach dem erfindungsgemäßen Verfahren hergestellten, erfindungsgemäßen MGS, das gegenüber dem Stand der Technik bei im Wesentlichen gleicher Masse eine wesentlich größer Teilchenoberfläche besitzt, erlaubt eine wirksame Reinigung von Körperflüssigkeiten, wie beispielsweise von Blut von nieder-, mittel - und hochmolekularen Endotoxinen und Exotoxinen (Ektotoxinen) ohne Störung der Fließeigenschaften des Blutes. Das nach dem erfindungsgemäßen Herstellungsverfahren hergestellte, erfindungsgemäße MGS ermöglicht eine Korrektur der Ferment- und Immunzusammensetzung von Körperflüssigkeiten, sowie die Vernichtung der pathogenen Mikroflora von Viren und Retroviren unter Anwendung einer kleineren Menge, i.e. einer kleineren Masse des erfindungsgemäßen MGS verglichen mit einem aus dem Stand der Technik bekannten MGS. Unter Berücksichtigung der Tatsache, dass eine Reinigung einer Körperflüssigkeit mithilfe des erfindungsgemäßen MGS durch Wechselwirkung dessen Oberfläche mit der zu berichtigenden Flüssigkeit erfolgt, so kann nachgewiesen werden, dass die wirksame Teilchenoberfläche des aus dem Stand der Technik bekannten Sorbens, dessen Abmessungen in Länge, Breite und Dicke im Wesentlichen identisch sind, unter der Voraussetzung einer im Wesentlichen gleich bleibenden Teilchenmasse wesentlich kleiner ist als die Teilchenoberfläche des erfindungsgemäßen Sorbens. Nachfolgend wird ein einem aus dem Stand der Technik bekannten Sorbens entsprechendes kugelartiges Teilchen betrachtet. Unter Verwendung der allgemein bekannten mathematischen Formeln, wird folgendes Ergebnis erhalten: Das Volumen V einer Kugel wird wie folgt berechnet: $V_{Kugel}$ = 4 π r³/3, mit r als dem Kugelradius. Die Oberfläche einer Kugel wird wie folgt berechnet: $S_{Kugel}$ = 4 π r², (1), entsprechend $S_{Kugel}$ = 3 $V_{Kugel}$/r.

Unter Berücksichtigung des Umstandes, dass die Masse eines Teilchens dessen Volumen proportional ist, und unter der Annahme, dass die nach dem oben beschriebenen, erfindungsgemäßen Verfahren zur Herstellung plattenförmiger Sorbensteilchen zunächst kugelförmig vorliegenden Sorbensteilchen in im Wesentlichen runde Platten umgeformt werden, sowie der Tatsache, dass das Volumen einer Scheibe/Platte V (Scheibe) = π R²δ und die Oberfläche S (Scheibenoberftäche) = π R² ist, R - Radius der Scheibe, δ - ihre Dicke, wobei bei δ = 0,1r (gemäß obiger Voraussetzung, dass die Dicke um 10-Grad geringer ist), erhalten wir, dass die Größe der Oberfläche S Oberfläche = V scneibe/01,r.

Unter der Voraussetzung, dass

$$V_{\text{Scheibe}} = V_{\text{Kugel}}$$

Und dass ihre Massen gleichen sind, erhält man

$$S_{\text{Scheibenoberfläche}} = 10\, V_{\text{Kugel}}/r. \qquad (2)$$

Unter Berücksichtigung, dass eine Scheibe zwei solche Flächen aufweist, und Einsetzung der Formel (1) in die Formel (2) erhalten wir

$$S_{\text{Gesamtoberfläche der Scheibe}} = 20\, S_{\text{Kugel}}/3.$$

Das Ergebnis bestätigt die oben angeführte Vermutung, dass die mit der Körperflüssigkeit wechselwirkende Oberfläche je Sorbensteilchens unter Verwendung des erfindungsgemäßen Sorbens wesentlich zunimmt. Hieraus ergibt sich, dass durch Verwendung des erfindungsgemäßen Sorbens die Menge des benötigten Sorbens und dementsprechend die Behandlungskosten vermindert werden können. Die Wirksamkeit des erfindungsgemäßen MGS, das mit Hilfe eines oben beschriebenen Verfahrens hergestellt wurde, wird durch die nachfolgenden Beispiele bestätigt:

Beispiel 1: Einem gattungslosen Hund mit einem Gewicht von 12 Kilo wurde eine Spritze mit (per os) 4,3 Gramm Barbital gegeben. Nach 45 Minuten betrug die Barbituratmenge im Blut 118 Mkg/Ml. Eine Korrektur des Blutes wurde in Form einer extrakorporalen Wiederherstellungsbehandlung des Blutes unter Verwendung eines entsprechenden Gerätes (UKBZ-1) durchgeführt. Das Blut des Tieres wurde batchweise je 10 MI. Ausgezogen. Die Batche wurden in jeweils gleichen Volumenverhältnissen mit einer Suspension des erfindungsgemäßen MGS in einer physiologischen Lösung vermischt, die Suspension enthielt (%) eines als Nickelplatten ausgeführte, zweilagig beschichtete Kerne mit einer Innenschicht aus Kohlenstoff und einer Außenschicht aus Dextran umfassenden MGS - 1,5, Koagulierschutzmittel (Heparin) - 0,015, die physiologische Lösung bildete den Rest. die Suspension wurde für 2 bis 3 Sekunden abgelagert, danach wurde dem Tier die Spritze mit dieser Suspension gegeben. Während der Behandlung wurde etwa 1 Liter Blut durchgeleitet.

Die Angaben vor und nach der Behandlung sind wie folgt:

| | | |
|---|---|---|
| Kreatinin (m Mol /Liter) | 1,45 | 1,10 |
| Kohlensäurediamid (Harnstoff) (m Mol/Liter) | 11,9 | 6,2 |
| Bilirubin (das gesamte) (m Mol /Liter) | 25,0 | 14,4 |
| Barbituraten (Mkg/MI) | 141,5 | 14,2 |

Außerdem wurde während der Behandlung eine Magenspülung durchgeführt und dem Tier 500 ml. Elektrodenflüssigkeitslösung und Glukose von 2 % intravenös eingespritzt. Nach der Behandlung hatte das Tier einen mittelschweren Zustand, die Reflexe waren lebhaft.

In den nachfolgenden Beispielen sind den Angaben der Wirksamkeit der erfindungsgemäßen Sorption selektive und funktionelle Eigenschaften der bekannten MGS gegenübergestellt, wie sie beispielsweise RU 2178313 entnehmbar sind. Die nach den durchgeführten Untersuchungen mit dem erfindungsgemäßen MGS erhaltenen Ergebnisse sind ebenfalls angeführt.

**Beispiel 2:** In ein Prüfglas mit 100 ml Blut eines gattungslosen Hundes werden 5 ml. Karbofoslösung zugesetzt. Die Karbofoskonzentration im Blut beträgt 0,015 Mkg/Ml. Die hergestellte Mischung wurde in zwei Teile geteilt und jedem Teil wurden 20 ml Suspension eines MGS zugegeben. In den ersten Teil wurde eine Suspension umfassend 1,0 g eines aus dem Stand der Technik bekannten MGS mit Eisenteilchen als Kerne und einer Beschichtung aus Siliziumoxid sowie eine physiologische Lösung eingespritzt. In den zweiten Teil wurde eine Suspension mit derselben Zusammensetzung eingespritzt, welche anstelle der oben genannten Kerne O,□,G plattenförmige Kerne enthält.

Beide so gewonnenen Mischungen werden anschließend getrennt voneinander vermischt und anschließend die Überniederschlagflüssigkeit in einem Zeitraum von 1,5 Minuten abgegossen. Der Niederschlag wird mittels eines Magneten gehalten. Die Karbofoskonzentration in der aus der ersten Mischung gewonnenen Überniederschlagflüssigkeit beträgt 0,002 Mkg/Ml, und in der aus der zweiten Mischung gewonnenen Überniederschlagflüssigkeit 0,012 Mkg/Ml.

**Beispiel 3**: In Prüfgläser mit je 20 ml Blutserum eines gattunglosen Hundes mit einer nachgebildeten Niereninsuffizienz (in der 1. Niere ist Kohlensäurediamid in einer Konzentration von 26,4 m Mol/Liter und in der 2. Niere Kohlensäurediamid mit einer Konzentration von 30,2 m Mol/Liter vorhanden) wurden 200 mg eines bekannten MGS mit einer durch Urease fermentierten Beschichtung aus Sefadex-10 (in die 1. Niere) und 30 mg eines erfindungsgemäßen MGS mit den den obengenannten Kernen gleichartig beschichteten Titanplatten (in die 2. Niere) eingebracht. Nach einer Lagerung der gewonnenen Mischungen für 5 Sekunden unter gleichzeitigem Schütteln und nach Beseitigung der Überniederschlagflüssigkeit im Magnetfeld beträgt die Konzentration des Kohlensäurediamids in der Überniederschlagflüssigkeit des ersten Prüfglas 10,7 m Mol/Liter und im zweiten Prüfglas beträgt sie 12,1 m Mol/Liter.

**Beispiel 4:** In zwei Prüfgläser mit je 20 ml Natriumsalzlösung der Phosphorsäure ($NaH_2 PO_4$) in Wasser werden in das erste Prüfglas 100 mg eines bekannten MGS mit einer Beschichtung aus Austauschharz, das mit Kationen (Polysaccharide mit $COOH^+$Grup.) modifiziert wurde, und in das zweite Prüfglas 10 mg eines erfindungsgemäßen MGS mit den den obengenannten Kernen gleichartig beschichteten Titanplatten eingebracht. Nach einer Vermischung der so hergestellten Mischungen durch Schütteln und nach Beseitigung der Überniederschlagflüssigkeit im Magnetfeld hat die Phosphatenkonzentration in der aus dem ersten Prüfglas gewonnenen Überniederschlagflüssigkeit um 57 % gegenüber der ursprünglichen Konzentration abgenommen. In der aus dem zweiten Prüfglas gewonnenen Überniederschlagflüssigkeit hat die Phosphatenkonzentration fast doppelt, genauer um 44,8 % gegenüber der ursprünglichen Konzentration abgenommen.

**Beispiel 5:** In zwei Prüfgläser mit je 20 ml wässrige Lösung Schwefelsäure wurden ins erste Prüfglas 100 mg eines bekannten MGS mit einer Beschichtung aus Austauschharz, das mit Anionenen (mit $NH_3$ x$^-$ Grup.) modifiziert wurde, und in das zweite Prüfglas 20 mg eines erfindungsgemäßen MGS mit den den obengenannten Kernen gleichartig beschichteten Eisennickelplatten eingebracht. Nach einer Vermischung der so hergestellten Mischungen durch Schütteln und nach Beseitigung der Überniederschlagflüssigkeit im Magnetfeld hat die Konzentration der Salze der Schwefelsäure in den aus den beiden Prüfgläsern gewonnenen Überniederschlagflüssigkeit praktisch gleichartig abgenommen. Im ersten Prüfglas hat die Konzentration um 72 % und im zweiten Prüfglas um 73,4 % gegenüber der ursprünglichen Konzentration abgenommen.

**Beispiel 6:** In zwei Prüfgläser mit je 20 ml Blut eines an einer chronischen Niereninsuffizienz leidenden Menschen wurden 100 mg eines bekannten MGS mit einer Beschichtung aus Dextran, das mit Zirkonkonsalz des Luminols modifiziert wurde, und in das zweite Prüfglas 30 mg eines erfindungsgemäßen MGS mit den den obengenannten Kernen gleichartig beschichteten Eisentitanplatten eingebracht. Nach einer Vermischung der so hergestellten Mischungen durch Schütteln und nach Beseitigung der Überniederschlagflüssigkeit im Magnetfeld beträgt die Konzentration der Salze der Phosphorsäure ($NaH_2PO_4$) in der aus dem ersten Prüfglas gewonnenen Überniederschlagflüssigkeit 0,07 mg/ml. In der aus dem zweiten Prüfglas gewonnenen Überniederschlagflüssigkeit beträgt die Konzentration der Salze der Phosphorsäure ($NaH_2PO_4$) in der aus dem ersten Prüfglas gewonnenen Überniederschlagflüssigkeit 0,021 mg/ml. Die ursprüngliche Konzentration dieses Salzes betrug dabei 0,61 mg/ml.

**Beispiel 7:** In zwei Prüfgläser mit je 10 ml Blutserum eines an einer chronischen Niereninsuffizienz leidenden Menschen wurden 50 mg eines bekannten MGS mit Kernen aus einer Eisennickellegierung und mit einer durch Urease modifizierten Beschichtung aus Sefadex, und in das zweite Prüfglas 10 mg eines erfindungsgemäßen MGS mit den den obengenannten Kernen gleichartig beschichteten plattenförmigen Kernen aus Eisennickellegierung eingebracht. Nach einer Lagerung der so hergestellten Mischungen für 10 Sekunden und nach dem Abgießen der Überniederschlagflüssigkeit im Anschluss an die Sorption hat die Kohlensäurediamidkonzentration in der aus dem ersten Prüfglas gewonnenen Überniederschlagflüssigkeit um 23 % gegenüber der ursprünglichen Konzentration abgenommen. In der aus dem zweiten Prüfglas gewonnenen Überniederschlagflüssigkeit hat die Kohlensäurediamidkonzentration um 35 % gegenüber der ursprünglichen Konzentration abgenommen.

**Beispiel 8:** In zwei Prüfgläser mit je 20 ml Blut eines an Sepsis leidenden Menschen wurden 150 mg eines bekannten MGS mit Kernen aus einer Eisennickellegierung und einer Beschichtung aus einer mit Oxazillin modifizierten Gelatine, und in das zweite Prüfglas 15 mg eines erfindungsgemäßen MGS mit den den obengenannten Kernen gleichartig beschichteten plattenförmigen Kernen aus Eisen, Titan und Tantal eingebracht. Nach einer zweiminütigen Vermischung der so hergestellten Mischungen durch Schütteln wurde die Überniederschlagflüssigkeit abgegossen und die festen Bestandteile in einem Magnetfeld festgehalten. Anschließend wurde Blut, das dem Kranken entnommen war, sowie das Blut das der Einwirkung des MGS ausgesetzt war, i.e. die Überniederschlagflüssigkeit aus den beiden Prüfgläsern, jeweils getrennt auf einen Nährboden aus Agar-Agar ausgebracht. Beim entnommenen Blut konnte eine Zunahme von Streptokokkuskolonien und von Staphylokokkus aureus beobachtet werden. Beim aus den Prüfgläsern entnommenen Blut konnte keine Zunahme von Streptokokkuskolonien beobachtet werden.

**Beispiel 9:** In zwei Prüfgläser mit je 10 ml Lympheplasma einer an Sepsis leidenden Frau wurden 100 mg eines bekannten MGS mit Kernen aus einer Eisennickellegierung und einer Beschichtung aus Dextran, und in das zweite Prüfglas 15 mg eines erfindungsgemäßen MGS mit den den obengenannten Kernen gleichartig beschichteten plattenförmigen Kernen aus Eisen, Titan und Tantal eingebracht. Nach Vermischen der so hergestellten Mischungen durch Schütteln wurde die Überniederschlagflüssigkeit im Magnetfeld abgegossen. Anschließend wurde Lympheplasma, das der Kranken entnommen war, sowie Lympheplasma, das der Einwirkung des MGS ausgesetzt war, i.e. die Überniederschlagflüssigkeit aus den beiden Prüfgläsern, jeweils getrennt auf einen Nährboden aus Agar-Agar ausgebracht. Beim entnommenen Blut konnte eine Zunahme von Streptokokkuskolonien beobachtet werden. Beim aus den Prüfgläsern entnommenen Blut konnte keine Zunahme von Streptokokkuskolonien beobachtet werden.

**Beispiel 10:** In zwei Prüfgläser mit je 5 ml Liquor, das mit Blut eines Menschen mit einer Schädelhirnverletzung gefärbt wurde, wurden 50 mg eines bekannten MGS mit Kernen aus Eisen und einer Beschichtung aus Siliziumdioxid, und in das zweite Prüfglas 15 mg eines erfindungsgemäßen MGS mit den den obengenannten Kernen gleichartig beschichteten plattenförmigen Kernen aus einer Eisentantallegierung eingebracht. Nach der Abscheidung hat das Liquor in den Prüfgläsern eine hellgelbe Farbe erworben.

Die Anwendbarkeit des erfindungsgemäßen MGS wird durch die oben beschriebenen Untersuchungen der Sorptionsfähigkeit bestätigt. Dabei wurden die unter Einsatz von vergleichbaren Varianten von aus dem Stand der Technik bekannten MGS erzielten Ergebnissen unter Verwendung deutlich geringerer Mengen an erfindungsgemäßem MGS erreicht.

**Patentansprüche**

1. Magnetisches Sorbens mit einer ein- oder zweilagigen Beschichtung oder ohne eine solche, umfassend einen ferromagnetischen Kern,
**dadurch gekennzeichnet,**
**dass** der Kern plattenförmig mit Flächenabmessungen von 500 bis 5000 $\mu$m und einer Dicke von 0,1 bis 1000 $\mu$m ausgebildet ist.

**2.** Magnetisches Sorbens nach Anspruch 1,
**dadurch gekennzeichnet,**
**dass** der Kern aus Eisen oder Eisenoxiden oder Nickel oder aus einer Eisennickellegierung oder einer Eisentitanlegierung oder einer Nickeltitanlegierung oder einer Eisentantallegierung oder einer Nickeltantallegierung oder einer Eisennickeltitanlegierung oder einer Eisennickeltantallegierung besteht.

**3.** Magnetisches Sorbens nach Anspruch 1 oder 2,
**gekennzeichnet durch**
eine einlagige Beschichtung aus Kohlenstoff oder Aluminiumoxiden oder Siliziumdioxid oder Zirkoniumdioxid oder Dextran oder Gelatine oder Albumin oder Polysacharid oder Austauschharzen.

**4.** Magnetisches Sorbens nach Anspruch 1 oder 2,
**dadurch gekennzeichnet,**
**dass** eine erste, dem Kern am nächsten liegende Lage einer zweilagigen Beschichtung aus Kohlenstoff oder Aluminiumoxiden oder Siliziumdioxid oder Zirkoniumdioxid besteht, und eine zweite, äußere Lage der Beschichtung aus Dextran oder Gelatine, oder Albumin oder Polysacharid oder aus Austauschharzen besteht.

**5.** Magnetisches Sorbens nach einem der Ansprüche 1 oder 3 bis 4,
**dadurch gekennzeichnet,**
**dass** die äußere Lage der Beschichtung ein Medikament oder einen medizinischen Wirkstoff enthält.

**6.** Verfahren nach einem der Ansprüche 1 bis 2,
**dadurch gekennzeichnet,**
**dass** zunächst ein pulverförmiges ferromagnetisches Material in einem kalten Plasma bei einer Temperatur von $10^4$x0,5 bis 5° C verdampft oder geschmolzen wird, anschließend das gewonnene dampfförmige oder als geschmolzene Teilchen der entsprechenden Metalle oder der entsprechenden Metallengemische vorliegende Produkt scharf abgekühlt und in einem Gasstrom kondensiert wird, danach das als Kristalle oder entsprechend als Mikroblöckchen des verwendeten ferromagnetischen Materials ausgeschiedene Produkt in ein Dispergens übertragen wird, das einen Stabilisator enthält, das Dispergens dann umgerührt und für 5 bis 15 Stunden bei einer Temperatur von 50 bis 90° C und beim Residualdruck von 1 bis 5 Millimeter Quecksilbersäule belassen wird, bis die Gasausscheidung aufhört, anschließend die Kristalle oder Mikroblöckchen bearbeitet und plattgedrückt werden, bis Platten der gewünschten Dicke erhalten werden, die Platten danach gespült und dann die schwachen Plattenteile abgetrennt werden, die Platten anschließend getrocknet und danach entsprechend der gewünschten Größe aussortiert werden, und dann auf den ausgeschiedenen Platten lageweise eine Beschichtung gebildet wird, und letztendlich das so gewonnene Produkt abgepackt und sterilisiert wird.

**7.** Verfahren nach Anspruch 6,
**dadurch gekennzeichnet,**
**dass** eine erste, dem Kern am nächsten liegende Lage der Beschichtung durch Wärmebehandlung der aussortierten Platten bei einer Temperatur von 1000 bis 1500° C in einem Inertgasstrom, der Mikroteilchen bestehend entweder aus Kohlenstoff oder aus Siliziumoxid, entweder aus Aluminiumoxid oder aus Zirkoniumoxid enthält, gebildet wird.

**8.** Verfahren nach Anspruch 6,
**dadurch gekennzeichnet,**
**dass** eine erste Lage der Beschichtung gebildet wird, indem auf eine Suspension aussortierter Platten in einer auf 30 bis 80° C temperierten wässrigen Lösung von Dextran oder von Gelatine, oder von Albumin oder von Stärke für einen Zeitraum von 1 bis 10 Minuten mithilfe einer Ultraschallquelle eingewirkt wird, diese Suspension dann bis auf eine Temperatur von 4 bis 10° C abgekühlt und der **dadurch** gewonnene Niederschlag mit Formalin abgegossen wird, der Niederschlag anschließend für einen Zeitraum von 10 bis 40 Minuten unter gleichzeitigem Umrühren im Formalin abgelagert wird und danach der Niederschlag bei einer Temperatur von 25 bis 50° C getrocknet und zermahlen wird und die so gewonnenen Sorbenskapseln in einem Magnetfeld aussortiert werden.

**9.** Verfahren nach Anspruch 6,
**dadurch gekennzeichnet,**
**dass** eine erste Lage der Beschichtung durch Zusetzen eines Austauschharzes in eine auf 40 bis 60° C erwärmte Suspension der aussortierten Platten in destilliertem Wasser gebildet wird, indem diese Suspension anschließend bis auf eine Temperatur von 15 bis 30° C abgekühlt, und wasserlösliche Salpetersäure ($HNO_2$) dazugegeben wird, die Suspension anschließend für 10 bis 15 Minuten abgelagert wird und dann bis zu einer Temperatur von 4 bis 10° C abgekühlt wird, wobei der entstehende Niederschlag ausgeschieden, in einer physiologischen Lösung gespült und in einer wässrigen Lösung umfassend ein Base-Salz-Gemisch aus $NH_4OH$ und $NH_4Cl$ gepuffert wird.

10. Verfahren nach Anspruch 6,
**dadurch gekennzeichnet,**
**dass** eine zweite Lage der Beschichtung unter Rühren gebildet wird, indem für einen Zeitraum von 1 bis 10 Minuten mit Ultraschall auf eine Suspension der ferromagnetischen, mit der ersten Lage der Beschichtung versehenen Kerne in einer erwärmten wässrigen Lösung von Dextran, Gelatine, Albumin oder Stärke eingewirkt wird, die Suspension dann bis auf eine Temperatur von 4 bis 10° C abgekühlt wird, wobei der auftretende Niederschlag mit Formalin abgegossen, und für einen Zeitraum von 10 bis 40 Minuten im Formalin unter gleichzeitigem Umrühren belassen wird, der Niederschlag dann bei einer Temperatur von 25 bis 50° C sorgfältig getrocknet und zermahlen wird und anschließend die gewonnenen, kapselartig zweilagig beschichteten, das Sorbens bildenden Kerne in einem Magnetfeld aussortiert werden.

11. Verfahren nach Anspruch 6,
**dadurch gekennzeichnet,**
**dass** eine zweite Lage der Beschichtung durch Zugabe eines Austauschharzes, beispielsweise von Amberlit, in die bis auf 40 bis 60° C erwärmte Suspension der ferromagnetischen, mit einer ersten Lage der Beschichtung versehenen Kerne in destilliertem Wasser gebildet wird, indem diese Suspension bis auf eine Temperatur von 15 bis 30° C abgekühlt und Albumin dazugegeben und vermischt wird, anschließend flüssige Salpetersäure ($HNO_2$) dazugegeben, und das Ganze für 10 bis 15 Minuten abgelagert wird, danach das Gemisch bis auf eine Temperatur von 4 bis 10° C abgekühlt wird, wobei sich ein Niederschlag ausscheidet, der aktiviert wird, indem dieser in einer Modifizierungsmittellösung für 1,5 bis 2 Stunden ablagert wird, der Niederschlag anschließend in einer physiologischen Lösung gespült und bis pH von 4,0 0,5 in der Wasserlösung der Basemischung $NH_4OH$ in Salz $NH_4Cl$ gepuffert wird.

12. Verfahren nach Anspruch 11,
**dadurch gekennzeichnet,**
**dass** als Modifizierungsmittel Natriumperiodat (Nal04) oder Glutaraldehyd in einer 3 bis 10 %igen wässrigen $Na_2SO_4$-Lösung eingesetzt wird.

13. Verfahren nach einem der Ansprüche 6 bis 11,
**dadurch gekennzeichnet,**
**dass** die äußere Lage der Beschichtung mit Antikörpern versehen wird, indem in eine wässrige Suspension ein magnetisches Sorbens mit einer ein- oder zweilagigen Beschichtung dazugegeben wird, dessen äußere Lage aus Sefadex oder aus Albumin besteht, der wässrigen Suspension ein Serum zugesetzt wird, welches Antikörper enthält, die für das zu sorbierende Antigen spezifisch sind, und diese Mischung in einer Pufferflüssigkeit mit pH von 6,5 bis 10 für einen Zeitraum von 1 bis 3 Stunden bei einer Temperatur von 15 bis 25° C abgelagert und vermischt wird, anschließend in diese Mischung Natriumborhydrid dazugegeben wird, die Mischung dann auf eine Temperatur von 4 bis 10° C abgekühlt, vermischt und wiederholt während 1 bis 3 Stunden abgelagert wird und dann der Niederschlag ausgezogen, gepuffert und getrocknet wird.

14. Verfahren nach einem der Ansprüche 6 bis 11,
**dadurch gekennzeichnet,**
**dass** die äußere Lage der Beschichtung während deren Herstellung mit einem Medikament oder mit einem medizinischen Wirkstoff modifiziert wird, indem eine das magnetische Sorbens mit ein- oder zweilagig beschichteten Kernen mit einer äußere Lage aus Dextran oder Gelatine umfassende Suspension in einer physiologischen Lösung bis zu einer Temperatur von 35 bis 70° C erwärmt wird, in diese Lösung ein vorzugsweise pulverförmiges Medikament oder ein pulverförmiger medizinischer Wirkstoff dazugegeben wird, die Mischung bei der oben genannten Temperatur für einen Zeitraum von 0,5 bis 2,5 Stunden abgelagert und vermischt wird und bis auf eine Temperatur von 4 bis 10° C abgekühlt wird, wobei anschließend die Überausscheidungsflüssigkeit in einem Magnetfeld abgegossen, und der Niederschlag unter fließendem destillierten Wassers gespült und später getrocknet wird.

15. Verfahren nach einem der Ansprüche 6 bis 11,
**dadurch gekennzeichnet,**
**dass** die äußere Lage der Beschichtung bei deren Erzeugung durch Vorlösung von Ureasekristalle in Makrodiol, beispielsweise in Dibenzo-18 Kraun 6 modifiziert wird, wobei diese Lösung mit einer das magnetische Sorbens mit einer Beschichtung und destilliertes Wasser umfassenden Suspension vermischt wird, die Mischung anschließend für einen Zeitraum von 2 bis 5 Stunden bei einer Temperatur von 25 bis 40°C abgelagert und bis auf eine Temperatur von 4 bis 10° C abgekühlt wird, danach Formaldehyd dazugegeben wird, und die Mischung wieder für 1 bis 3 Stunden abgelagert wird und anschließend die Überausscheidungsflüssigkeit in einem Magnetfeldes abgegossen und der Niederschlag getrocknet wird.

**16.** Verfahren nach einem der Ansprüche 6 bis 11,
**dadurch gekennzeichnet,**
**dass** die äußere Lage der Beschichtung modifiziert wird, indem eine ein magnetisches Sorbens mit einer äußeren Lage aus Dextran enthaltende wässrige Suspension bis zu einer Temperatur von 40 bis 70° C erwärmt wird, die Suspension anschließend mit einem Zirkoniumsalzpulver, beispielsweise mit einem entsprechenden Phthalhydrozidsalz, vermischt wird, indem auf diese Mischung mit Ultraschall mit einer Schallintensität von 50 bis 120 Volt/Zentimeter für einen Zeitraum von 1 bis 10 Minuten eingewirkt wird, danach die gewonnene Mischung bis auf eine Temperatur von 4 bis 10° C abgekühlt wird und Formaldehyd dazugegeben wird, und anschließend für 1 bis 3 Stunden abgelagert und umgerührt wird, und dann die Überausscheidungsflüssigkeit in einem Magnetfeldes abgegossen und die Ausscheidung getrocknet wird.

# EP 1 683 572 A1

**INTERNATIONAL SEARCH REPORT**

| International application No. |
| --- |
| PCT/RU 2004/000366 |

**A. CLASSIFICATION OF SUBJECT MATTER**

B01J 20/02, 20/06, 20/08, 20/10, 20/24, 20/26, 20/30

According to International Patent Classification (IPC) or to both national classification and IPC

**B. FIELDS SEARCHED**

Minimum documentation searched (classification system followed by classification symbols)

B01J 20/00-20/30

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y<br>A | RU 2178313 c1 (KUTUSHOV MIKHAIL VLADIMIROVICH) 20.01.2002, the claims | 1-5<br>6-16 |
| Y | RU 2065606 C1 (SKOPINTSEV JURY PETROVICH et al) 20.08.1996, column 4, paragraph 2 | 1-5 |
| A | RU 2109522 C1 (KUTUSHOV MIKHAIL VLADIMIROVICH et al) 27.04.1998 | 1-16 |
| A | SU 1589327 A1 (KLINIKA KHIRURGICHESKIKH BOLEZNEI INSTITUTA KIBERNETIKI AN GSSR et al) 30.08.1990 | 1-16 |
| A | US 6616623 B1 (IDIALIZA LTD.) 09. 09. 2003 | 1-16 |
| A | US 6136428 A (IMATION CORP.) 24. 10. 2000 | 1-16 |

☐ Further documents are listed in the continuation of Box C.   ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 20 December 2004 (20.12.2004) | 20 January 2005 (20.01.2005) |

| Name and mailing address of the ISA/ | Authorized officer |
| --- | --- |
| RU | |
| Facsimile No. | Telephone No. |

Form PCT/ISA/210 (second sheet) (July 1992)

13